# EUROPEAN PATENT SPECIFICATION

(11) **EP 3 508 123 B1**
(45) Date of publication and mention of the grant of the patent: **20.09.2023**
(21) Application number: 17846079.6
(22) Date of filing: 08.08.2017
(51) Int. Cl.: A61B 5/1455, A61B 10/00, A61B 5/00

(54) **METHOD OF EVALUATING THERAPEUTIC EFFECT OF DRUG IN CANCER PATIENT, METHOD OF GENERATING EVALUATION DATA OF THERAPEUTIC EFFECT, THERAPEUTIC EFFECT EVALUATION DEVICE, AND THERAPEUTIC EFFECT EVALUATION PROGRAM**
VERFAHREN ZUR BEURTEILUNG DER THERAPEUTISCHEN WIRKUNG EINES ARZNEIMITTELS BEI EINEM KREBSPATIENTEN, VERFAHREN ZUR GENERIERUNG VON DATEN ZUR BEURTEILUNG DER THERAPEUTISCHEN WIRKUNG, VORRICHTUNG ZUR BEURTEILUNG DER THERAPEUTISCHEN WIRKUNG UND PROGRAMM ZUR BEURTEILUNG DER THERAPEUTISCHEN WIRKUNG
PROCÉDÉ D'ÉVALUATION DE L'EFFET THÉRAPEUTIQUE D'UN MÉDICAMENT CHEZ UN PATIENT CANCÉREUX, PROCÉDÉ DE PRODUCTION DE DONNÉES D'ÉVALUATION DE L'EFFET THÉRAPEUTIQUE, DISPOSITIF D'ÉVALUATION DE L'EFFET THÉRAPEUTIQUE, ET PROGRAMME D'ÉVALUATION DE L'EFFET THÉRAPEUTIQUE

(30) Priority: 05.09.2016 JP 2016172876
(43) Date of publication of application: 10.07.2019
(73) Proprietor: Saitama Medical University, Iruma-gun, Saitama 350-0495 (JP); Hamamatsu Photonics K.K., Hamamatsu-shi, Shizuoka 435-8558 (JP)
(72) Inventor: UEDA Shigeto, Iruma-gun Saitama 350-0495 (JP); SAEKI Toshiaki, Iruma-gun Saitama 350-0495 (JP); UEDA Yukio, Hamamatsu-shi Shizuoka 435-8558 (JP)
(74) Representative: Hoffmann Eitle
(86) International application number: PCT/JP2017/028807
(87) International publication number: WO 2018/043070

(56) References cited:
- WO-A1-2015/166110
- JP-A- 2010 524 554
- S. UEDA ET AL: "Near-Infrared Diffuse Optical Imaging for Early Prediction of Breast Cancer Response to Neoadjuvant Chemotherapy: A Comparative Study Using 18F-FDG PET/CT", THE JOURNAL OF NUCLEAR MEDICINE, vol. 57, no. 8, 1 August 2016 (2016-08-01) , pages 1189-1195, XP055674978, US ISSN: 0161-5505, DOI: 10.2967/jnumed.115.167320
- ALBERT E. CERUSSI ET AL: "Diffuse optical spectroscopic imaging correlates with final pathological response in breast cancer neoadjuvant chemotherapy", ROYAL SOCIETY OF LONDON. PHILOSOPHICAL TRANSACTIONS.MATHEMATICAL, PHYSICAL AND ENGINEERING SCIENCES, vol. 369, no. 1955, 28 November 2011 (2011-11-28), pages 4512-4530, XP055675072, GB ISSN: 1364-503X, DOI: 10.1098/rsta.2011.0279
- YOSHIZAWA NOBUKO ET AL: "Effect of the chest wall on the measurement of hemoglobin concentrations by near-infrared time-resolved spectroscopy in normal breast and cancer", BREAST CANCER, JAPANESE BREAST CANCER SOCIETY, TOKYO, JP, vol. 23, no. 6, 16 October 2015 (2015-10-16), pages 844-850, XP036085675, ISSN: 1340-6868, DOI: 10.1007/S12282-015-0650-7 [retrieved on 2015-10-16]

## Description

### Technical Field

The present disclosure relates to a method of evaluating a therapeutic effect of a drug in a cancer patient, a method of generating evaluation data of a therapeutic effect, a therapeutic effect evaluation device, and a therapeutic effect evaluation program.

### Background Art

Drug therapies for cancer patients using anticancer agents or the like greatly contribute to an improvement in survival rate of cancer patients, while it is known that some cancers are refractory to these drugs. Generally, in the drug therapies for cancers, a cycle is repeated several times, in which a drug is administered for a predetermined period, a predetermined period of drug holidays is disposed, and then the drug is again administered, and in some cases, several months are needed to evaluate the therapeutic effect of the drug. If the evaluation of the therapeutic effect takes a long time as described above, those cancers refractory to the drug may unfortunately progress in some cases. For this reason, it is desired to predict the presence/absence of the therapeutic effect of the drug at earlier stages of the start of the drug therapy, and to perform treatments, for example, to switch early the current therapy to another therapy in the cancers on which the drug exerts a small therapeutic effect.

As a method of evaluating the therapeutic effect of a drug therapy relatively in a short time from the start thereof, for example, Non Patent Literatures 1 and 2 disclose evaluation of an therapeutic effect by measuring a hemoglobin concentration in a lesion site of a breast cancer patient before and after administration of a drug, performing mapping thereof, and evaluating the therapeutic effect based on the obtained optical mammographic image. These methods have advantages in that there is few invasion to the cancer patients because the lesion site is irradiated with near-infrared light.

### Citation List

### Non Patent Literature

Non Patent Literature 1: Technology in Cancer Research and Treatment, 2011, 10(5), 393.
Non Patent Literature 2: BMC Cancer 2013 13, 514.

### Summary of Invention

### Technical Problem

However, while the methods according to Non Patent Literatures 1 and 2 can evaluate the therapeutic effects on cancers present on body surface tissues, measurement of the precise hemoglobin concentration due to influences of myoglobin present in muscular tissues in cancers present in deeper tissues in the body is difficult, and there is a problem that the evaluation of the therapeutic effect is difficult.

An object of an embodiment is to provide a method of evaluating a therapeutic effect in a cancer patient by a drug which can simply evaluate the therapeutic effect also on cancers present in deeper tissues in the body in a short time without invasion to the cancer patient, a method of generating evaluation data of a therapeutic effect, a therapeutic effect evaluation device, and a therapeutic effect evaluation program.

### Solution to Problem

The present invention provides an evaluation device as defined in claim 1 and an evaluation program as defined in claim 8. Preferred embodiments of the invention are defined in the dependent claims.

In the following description reference is made to embodiments of a method. The referred method(s) disclose details of the operation performed by the device or evaluation program. The method(s) as such do not form part of the claimed invention.

The evaluation method according to one embodiment is a method of evaluating a therapeutic effect of a drug in a cancer patient, comprising: a comparison step of comparing a first absolute hemoglobin concentration in a sternum portion of the cancer patient before administration of the drug and a second absolute hemoglobin concentration in the sternum portion in the cancer patient after administration of the drug; and an evaluation step of evaluating the therapeutic effect of the drug in the cancer patient based on the result of comparison performed through the comparison step. According to such an evaluation method, the therapeutic effect can be simply evaluated also in cancers present in deeper tissues in the body in a short time without invasion to cancer patients.

Moreover, in one embodiment, it may be evaluated in the evaluation step that there is a therapeutic effect of the drug if the second absolute hemoglobin concentration is lower than the first absolute hemoglobin concentration. Moreover, in one embodiment, it may be evaluated in the evaluation step that there is no therapeutic effect of the drug if the second absolute hemoglobin concentration is equal to or higher than the first absolute hemoglobin concentration. Thereby, the therapeutic effect can be more simply evaluated.

Furthermore, in one embodiment, the first absolute hemoglobin concentration may be a statistical value of data of a plurality of absolute hemoglobin concentrations measured in the sternum portion of the cancer patient before administration of the drug, and the second absolute hemoglobin concentration may be a statistical value of data of a plurality of absolute hemoglobin concentrations measured in the sternum portion of the cancer patient after administration of the drug. Thereby, more precise evaluation of the therapeutic effect becomes possible.

Furthermore, in one embodiment, the method may further comprise a comparison step of comparing a third absolute hemoglobin concentration in a lesion site of the cancer patient before administration of the drug and a fourth absolute hemoglobin concentration in the lesion site of the cancer patient after administration of the drug. Moreover, in one embodiment, it may be evaluated in the evaluation step that there is a therapeutic effect of the drug if the second absolute hemoglobin concentration is lower than the first absolute hemoglobin concentration and the fourth absolute hemoglobin concentration is lower than the third absolute hemoglobin concentration. In these embodiments, the therapeutic effect can be more simply evaluated.

Moreover, in an evaluation method according to one embodiment, the cancer patient may be a breast cancer patient.

Moreover, a method of generating evaluation data according to one embodiment is a method of generating evaluation data of a therapeutic effect of a drug in a cancer patient, comprising: a comparison step of comparing a first absolute hemoglobin concentration in a sternum portion of the cancer patient before administration of the drug and a second absolute hemoglobin concentration in the sternum portion of the cancer patient after administration of the drug; an evaluation step of evaluating a therapeutic effect of the drug in the cancer patient based on the result of comparison performed through the comparison step; and a generation step of generating evaluation data to evaluate the therapeutic effect of the drug in the cancer patient based on the result of evaluation performed through the evaluation step. According to the evaluation data generated by such a method, the therapeutic effect on cancers present in deeper tissues in the body can also be evaluated.

The evaluation device according to one embodiment is an evaluation device of a therapeutic effect of a drug in a cancer patient, the evaluation device comprising: a data obtainer obtaining data of an absolute hemoglobin concentration in a sternum portion of the cancer patient before administration of the drug and data of an absolute hemoglobin concentration in the sternum portion of the cancer patient after administration of the drug; a data generator generating data of a first absolute hemoglobin concentration from the data of the absolute hemoglobin concentration in the sternum portion of the cancer patient before administration of the drug obtained by the data obtainer, and generating data of a second absolute hemoglobin concentration from the data of the absolute hemoglobin concentration in the sternum portion of the cancer patient after administration of the drug obtained by the data obtainer; and a data comparator comparing the data of the first absolute hemoglobin concentration and the data of the second absolute hemoglobin concentration. According to such an evaluation device, the therapeutic effect can also be simply evaluated on cancers present in deeper tissues in the body in a short time without invasion to cancer patients.

Furthermore, in one embodiment, the evaluation device of a therapeutic effect of a drug in a cancer patient may further comprise a data evaluator evaluating a therapeutic effect of the drug in the cancer patient based on the result of comparison performed by the data comparator. Moreover, in one embodiment, the data evaluator may evaluate that there is a therapeutic effect of the drug if the second absolute hemoglobin concentration is lower than the first absolute hemoglobin concentration. Moreover, in one embodiment, the data evaluator may evaluate that there is no therapeutic effect of the drug if the second absolute hemoglobin concentration is equal to or higher than the first absolute hemoglobin concentration. In these embodiments, the therapeutic effect can be more simply evaluated.

Furthermore, in one embodiment, the data generator may generate a statistical value of data of a plurality of absolute hemoglobin concentrations as the data of the first absolute hemoglobin concentration, and/or generate a statistical value of data of a plurality of absolute hemoglobin concentrations as the data of the second absolute hemoglobin concentration. Thereby, more precise evaluation of the therapeutic effect becomes possible.

Furthermore, in one embodiment, the data obtainer may obtain data of the absolute hemoglobin concentration in a lesion site of the cancer patient before administration of the drug and data of the absolute hemoglobin concentration in the lesion site of the cancer patient after administration of the drug, the data generator may generate data of a third absolute hemoglobin concentration from the data of the absolute hemoglobin concentration in the lesion site of the cancer patient before administration of the drug obtained by the data obtainer and data of a fourth absolute hemoglobin concentration from the data of the absolute hemoglobin concentration in the lesion site of the cancer patient after administration of the drug obtained by the data obtainer, and the data comparator may compare the data of the third absolute hemoglobin concentration and the data of the fourth absolute hemoglobin concentration.

Moreover, in one embodiment, the evaluator may evaluate that there is a therapeutic effect of the drug on the cancer patient if the second absolute hemoglobin concentration is lower than the first absolute hemoglobin concentration and the fourth absolute hemoglobin concentration is lower than the third absolute hemoglobin concentration. In these embodiments, the therapeutic effect can be more simply evaluated.

The evaluation program according to one embodiment is a program for evaluating a therapeutic effect of a drug in a cancer patient, the program causing a computer to execute: data obtaining processing to obtain data of an absolute hemoglobin concentration in a sternum portion in the cancer patient before administration of the drug and data of an absolute hemoglobin concentration in a sternum portion of the cancer patient after administration of the drug; data generation processing to generate data of a first absolute hemoglobin concentration from the data of the absolute hemoglobin concentration in the sternum portion of the cancer patient before administration of the drug obtained by the data obtainer, and to generate data of a second absolute hemoglobin concentration from the data of the absolute hemoglobin concentration in the sternum portion of the cancer patient after administration of the drug obtained by the data obtainer; and data comparison processing to compare the data of the first absolute hemoglobin concentration and the data of the second absolute hemoglobin concentration.

### Advantageous Effect of Invention

According to the evaluation method, the method of generating evaluation data, the evaluation device, and the evaluation program according to one embodiment, the therapeutic effect can be simply evaluated also on cancers present in deeper tissues in the body in a short time without invasion to cancer patients.

### Brief Description of Drawings

Figure 1 is a diagram schematically illustrating measurement of an absolute hemoglobin concentration with an evaluation device according to one embodiment.
Figure 2 is a schematic diagram illustrating a hardware-like configuration of the evaluation device according to one embodiment.
Figure 3 is a schematic diagram illustrating a functional configuration of the evaluation device according to one embodiment.
Figure 4 is a flowchart of an evaluation method according to one embodiment.
Figure 5 is a diagram illustrating a site where an absolute hemoglobin concentration was measured in Example 1.
Figure 6 is a graph showing ROC curves created in Reference Example 1.
Figure 7 is a graph showing ROC curves created in Reference Example 2.

### Description of Embodiments

Hereinafter, embodiments of the evaluation method, the method of generating evaluation data, the evaluation device, and the evaluation program will be described in detail with reference to the accompanied drawings. In the description of the drawings, the same reference numerals will be given to the same components, and the duplication of the description will be omitted.

### [Method of evaluating therapeutic effect of drug in cancer patient]

### (Comparison and evaluation of absolute hemoglobin concentrations in sternum portion)

One evaluation method according to the present embodiment is a method of evaluating a therapeutic effect of a drug in a cancer patient, the method comprising a comparison step of comparing a first absolute hemoglobin concentration in a sternum portion of the cancer patient before administration of the drug and a second absolute hemoglobin concentration in the sternum portion of the cancer patient after administration of the drug; and an evaluation step of evaluating the therapeutic effect of the drug in the cancer patient based on the result of comparison performed through the comparison step.

A "sternum portion (sternum site)" herein refers to a flat site located in the center of the front surface of the rib cage including an elongate bone. Because in the sternum portion, a large amount of bone marrows is present but there is a relatively small amount of muscular tissues, the absolute hemoglobin concentration can be non-invasively measured from the body surface with high precision. The present inventors have found that changes in absolute hemoglobin concentration in cancer patients before and after administration of a drug are observed not only in lesion sites but also in the sternum portion. About the changes in absolute hemoglobin concentration in the sternum portion, the present inventors infer that the absolute hemoglobin concentration in the sternum portion before and after administration of the drug changes in corporation with the absolute hemoglobin concentration in the lesion site, because the drug to be administered to cancer patients affects not only the lesion sites but also the entire bodies thereof and because cancer cells are also present in the bone marrows. Accordingly, in the evaluation method according to the present embodiment, the therapeutic effect can be evaluated about any cancers without limitation, and the therapeutic effects of patients suffering from cancers such as breast cancer, uterus cancer, ovarian cancer, esophagus cancer, stomach cancer, colorectal cancer, liver cancer, pancreatic cancer, brain malignant tumors, kidney cancer, lung cancer, prostate cancer, testicular cancer, bladder cancer, skin cancer, and leukemia can be evaluated. Among these, the evaluation method according to the present embodiment is suitably used in order to evaluate the therapeutic effect on breast cancer patients having lesion sites near the sternum portion.

The "drug" herein refers to a drug for treating cancers, and examples thereof include anticancer agents and hormonal agents. The drug to be administered may be composed of a single drug alone, or may be composed of a combination of two or more drugs. Moreover, the administration of the drug may be oral administration, or may be parenteral administration.

The "absolute hemoglobin concentration" herein refers to the total value of the absolute value of the concentration of hemoglobin bound to oxygen (oxygenated hemoglobin (HbO₂)) and the absolute value of the concentration of hemoglobin not bound to oxygen (deoxygenated hemoglobin (Hb)). It should be noted that the "absolute hemoglobin concentration" is not limited to this, and the absolute value of the concentration of oxygenated hemoglobin or the absolute value of the concentration of deoxygenated hemoglobin may be defined as the "absolute hemoglobin concentration".

In the comparison step, the first absolute hemoglobin concentration in the sternum portion of the cancer patient before administration of the drug is compared to the second absolute hemoglobin concentration in the sternum portion of the cancer patient after administration of the drug.

Generally, in the drug therapies for cancers, a cycle is repeated several times, in which a drug is administered for a predetermined period, a predetermined period of drug holidays is disposed, and then the drug is again administered. Accordingly, the second absolute hemoglobin concentration may be the absolute hemoglobin concentration after the first administration of the drug (after cycle 1), may be the absolute hemoglobin concentration after the second administration of the drug (after cycle 2), or may be the absolute hemoglobin concentration after the third or later administration of the drug (after cycle 3 or later).

In the evaluation step, the therapeutic effect of the drug in the cancer patient is evaluated based on the result obtained in the comparison step. Specifically, it is evaluated that there is a therapeutic effect of the drug if the second absolute hemoglobin concentration is lower than the first absolute hemoglobin concentration. In contrast, it is evaluated that there is no therapeutic effect of the drug if the second absolute hemoglobin concentration is equal to or higher than the first absolute hemoglobin concentration (that is, the second absolute hemoglobin concentration is equal to the first absolute hemoglobin concentration or the second absolute hemoglobin concentration is higher than the first absolute hemoglobin concentration). During evaluating that there is a therapeutic effect of the drug if the second absolute hemoglobin concentration is lower than the first absolute hemoglobin concentration, a reduction rate of the second absolute hemoglobin concentration to the first absolute hemoglobin concentration is preferably 3% or more, more preferably 5% or more, still more preferably 7% or more.

In the evaluation method according to the present embodiment, the first absolute hemoglobin concentration and the second absolute hemoglobin concentration may be 1) the absolute hemoglobin concentration measured one time in a single site of the sternum portion of the cancer patient, may be 2) a statistical value, such as an average or median of the data of absolute hemoglobin concentrations measured several times in a single site of the sternum portion of the cancer patient, or may be 3) a statistical value, such as an average or median of the data of the absolute hemoglobin concentrations measured one or several times in a plurality of different sites of the sternum portion in the cancer patient. If the first absolute hemoglobin concentration and the second absolute hemoglobin concentration are 2) or 3) above, more precise evaluation of the therapeutic effect becomes possible.

### (Comparison and evaluation of absolute hemoglobin concentration in lesion site)

The evaluation method according to the present embodiment comprises a comparison step of comparing a third absolute hemoglobin concentration in a lesion site of the cancer patient before administration of the drug and a fourth absolute hemoglobin concentration in the lesion site of the cancer patient after administration of the drug. By combining the evaluation by comparison of the absolute hemoglobin concentrations in the sternum portion with the evaluation by comparison of the absolute hemoglobin concentrations in the lesion site, more precise evaluation of the therapeutic effect becomes possible.

The "lesion site (pathological lesion site)" herein refers to a site where pathological changes are occurring due to the presence of cancer cells.

In the comparison step, the third absolute hemoglobin concentration in the lesion site of the cancer patient before administration of the drug is compared to the fourth absolute hemoglobin concentration in the lesion site of the cancer patient after administration of the drug.

Generally, in the drug therapies for cancers, a cycle is repeated several times, in which a drug is administered for a predetermined period, a predetermined period of drug holidays is disposed, and then the drug is again administered. Accordingly, the fourth absolute hemoglobin concentration may be the absolute hemoglobin concentration after the first administration of the drug (after cycle 1), may be the absolute hemoglobin concentration after the second administration of the drug (after cycle 2), or may be the absolute hemoglobin concentration after third or later administration of the drug (after cycle 3 or later).

In the evaluation step, the therapeutic effect of the drug in the cancer patient is evaluated based on the result obtained in the comparison step. Specifically, it is evaluated that there is a therapeutic effect of the drug if the second absolute hemoglobin concentration is lower than the first absolute hemoglobin concentration and the fourth absolute hemoglobin concentration is lower than the third absolute hemoglobin concentration. In contrast, it is evaluated that there is no therapeutic effect of the drug if the second absolute hemoglobin concentration is equal to or higher than the first absolute hemoglobin concentration and the fourth absolute hemoglobin concentration is equal to or higher than the third absolute hemoglobin concentration (that is, the second absolute hemoglobin concentration is equal to the first absolute hemoglobin concentration or the second absolute hemoglobin concentration is higher than the first absolute hemoglobin concentration, and the fourth absolute hemoglobin concentration is equal to the third absolute hemoglobin concentration or the fourth absolute hemoglobin concentration is higher than the third absolute hemoglobin concentration). It should be noted that for example, the therapeutic effect can be evaluated with a further combination with other observations if the second absolute hemoglobin concentration is lower than the first absolute hemoglobin concentration and the fourth absolute hemoglobin concentration is equal to or higher than the third absolute hemoglobin concentration (that is, the fourth absolute hemoglobin concentration is equal to the third absolute hemoglobin concentration or the fourth absolute hemoglobin concentration is higher than the third absolute hemoglobin concentration), or if the second absolute hemoglobin concentration is equal to or higher than the first absolute hemoglobin concentration (that is, the second absolute hemoglobin concentration is equal to the first absolute hemoglobin concentration or the second absolute hemoglobin concentration is higher than the first absolute hemoglobin concentration) and the fourth absolute hemoglobin concentration is lower than the third absolute hemoglobin concentration. During evaluating that there is a therapeutic effect of the drug if the second absolute hemoglobin concentration is lower than the first absolute hemoglobin concentration and the fourth absolute hemoglobin concentration is lower than the third absolute hemoglobin concentration, the reduction rate of the second absolute hemoglobin concentration to the first absolute hemoglobin concentration is preferably 3% or more, more preferably 5% or more, still more preferably 7% or more. Moreover, the reduction rate of the fourth absolute hemoglobin concentration to the third absolute hemoglobin concentration is preferably 23% or more, more preferably 30% or more.

In the evaluation method according to the present embodiment, the third absolute hemoglobin concentration and the fourth absolute hemoglobin concentration may be 1) an absolute hemoglobin concentration measured one time at a single site of the lesion site of the cancer patient, may be 2) a statistical value such as an average or a median of the data of the absolute hemoglobin concentrations measured several times in a single site of the lesion site in the cancer patient, or may be 3) a statistical value such as an average or a median of the data of absolute hemoglobin concentrations measured one or several times at a plurality of different sites of the lesion site of the cancer patient. If the third absolute hemoglobin concentration and the fourth absolute hemoglobin concentration may be 2) or 3) above, more precise evaluation of the therapeutic effect becomes possible.

The evaluation method according to the present embodiment may further comprise a first obtaining step of obtaining a first absolute hemoglobin concentration in the sternum portion of the cancer patient before administration of the drug, and a second obtaining step of obtaining a second absolute hemoglobin concentration in the sternum portion of the cancer patient after administration of the drug.

In the first obtaining step and the second obtaining step, the first absolute hemoglobin concentration in the sternum portion of the cancer patient before administration of the drug and the second absolute hemoglobin concentration in the sternum portion of the cancer patient after administration of the drug are obtained, respectively. The second obtaining step may be a step of obtaining the absolute hemoglobin concentration after the first administration of the drug (after cycle 1), may be a step of obtaining the absolute hemoglobin concentration after the second administration of the drug (after cycle 2), or may be a step of obtaining the absolute hemoglobin concentration after third or later administration of the drug (after cycle 3 or later).

The absolute hemoglobin concentration can be measured by time resolved spectroscopy or phase modulation spectroscopy, for example. The absolute hemoglobin concentration can be measured by any other method than time resolved spectroscopy or phase modulation spectroscopy without limitation.

In time resolved spectroscopy (TRS; Time Resolved Spectroscopy), pulsed light having an extremely short time width of several picoseconds is caused to enter the sternum portion, and the time resolved waveform of the light detected on the body surface is detected with an ultra-high speed photodetector. After the values of the absorption coefficient and the reduced scattering coefficient are calculated by fitting the time response properties of the detected light to the time response properties based on the optical diffusion theory, the absolute value of the oxygenated hemoglobin concentration and the absolute value of the deoxygenated hemoglobin concentration are calculated by the method of least squares. The absolute hemoglobin concentration, which is the total value of these values, is then calculated.

In phase modulation spectroscopy (PMS; Phase Modulation Spectroscopy), light modulated into a sine wave of about 100 MHz is caused to enter the sternum portion, the absolute value of the oxygenated hemoglobin concentration and the absolute value of the deoxygenated hemoglobin concentration are calculated from changes in phase and amplitude of the light detected on the body surface. The absolute hemoglobin concentration, which is the total value of these values, is then calculated.

In the evaluation method according to the present embodiment, the first obtaining step and the second obtaining step may be 1) a step of obtaining the absolute hemoglobin concentration one time from a single site of the sternum portion of the cancer patient, may be 2) a step of obtaining the absolute hemoglobin concentrations several times at a single site of the sternum portion of the cancer patient, or may be 3) a step of obtaining the absolute hemoglobin concentrations one or several times from a plurality of different sites of the sternum portion of the cancer patient. Accordingly, in the evaluation method according to the present embodiment, the first absolute hemoglobin concentration and the second absolute hemoglobin concentration may be 1) an absolute hemoglobin concentration measured one time at a single site of the sternum portion of the cancer patient, may be 2) a statistical value such as an average or a median of the data of the absolute hemoglobin concentrations measured several times at a single site of the sternum portion of the cancer patient, or may be 3) a statistical value such as an average or a median of the data of the absolute hemoglobin concentrations measured one or several times at a plurality of different sites of the sternum portion of the cancer patient.

The evaluation method according to the present embodiment may further comprise a third obtaining step of obtaining an absolute hemoglobin concentration in the lesion site of the cancer patient before administration of the drug, and a fourth obtaining step of obtaining an absolute hemoglobin concentration in the lesion site of the cancer patient after administration of the drug. The third obtaining step and the fourth obtaining step may be performed by the same method as in the first obtaining step and the second obtaining step.

The evaluation method according to the present embodiment can be also considered as a data collecting method for evaluating the therapeutic effect of a drug in a cancer patient, the method comprising a first obtaining step of obtaining a first absolute hemoglobin concentration in a sternum portion of a cancer patient before administration of the drug, and a second obtaining step of obtaining a second absolute hemoglobin concentration in the sternum portion of the cancer patient after administration of the drug. The data collecting method may further comprise a comparison step of comparing the first absolute hemoglobin concentration and the second absolute hemoglobin concentration. For example, it is evaluated that there is a therapeutic effect of the drug if the second absolute hemoglobin concentration is lower than the first absolute hemoglobin concentration. Alternatively, it is evaluated that there is no therapeutic effect of the drug if the second absolute hemoglobin concentration is equal to or higher than the first absolute hemoglobin concentration.

### [Method of generating evaluation data of therapeutic effect of drug in cancer patient]

The method of generating evaluation data according to the present embodiment comprises a comparison step of comparing a first absolute hemoglobin concentration in a sternum portion of a cancer patient before administration of the drug and a second absolute hemoglobin concentration in the sternum portion of the cancer patient after administration of the drug; an evaluation step of evaluating the therapeutic effect of the drug in the cancer patient based on the result of comparison performed through the comparison step; and a generation step of generating evaluation data to evaluate the therapeutic effect of the drug in the cancer patient based on the result of evaluation performed through the evaluation step. The generated evaluation data is provided to the cancer patient, a medical profession, or a medical institution through a network or the like.

### [Therapeutic effect evaluation device of drug in cancer patient]

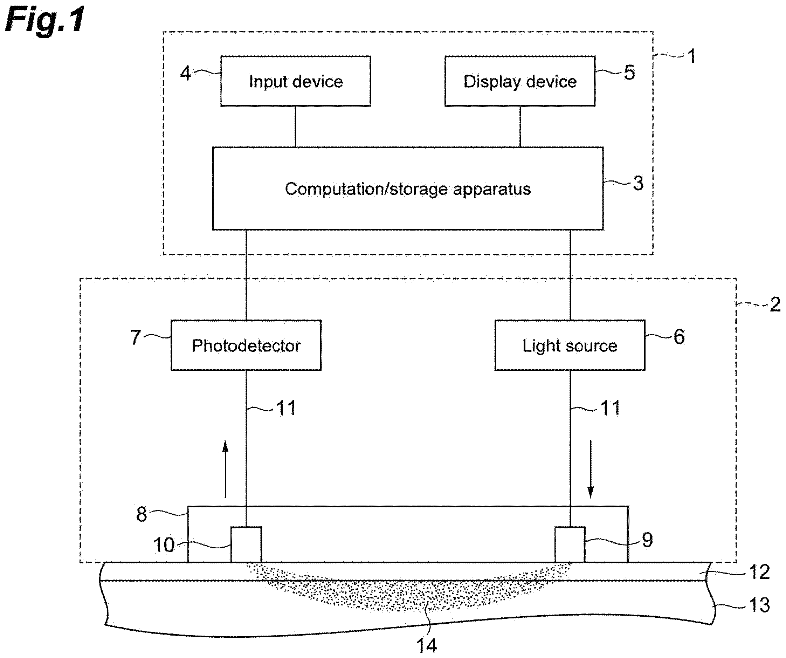

Figure 1 is a diagram schematically illustrating the measurement of the absolute hemoglobin concentration according to an evaluation device 1 according to one embodiment. The evaluation device 1 according to one embodiment is composed of a computation/storage apparatus (computer) 3, an input device 4, and a display device (display) 5, and can be used in a combination with an optical measuring apparatus 2 comprising a light source 6, a photodetector 7, and a probe 8.

The computation/storage apparatus 3 is connected to the light source 6 and the photodetector 7 so as to be capable of communicating with each other, or is electrically connected to the light source 6 and the photodetector 7. The computation/storage apparatus 3 controls the output of the measured light from the light source 6 and obtains the detected signals from the photodetector 7. Moreover, the computation/storage apparatus 3 obtains the detected signals output from the photodetector 7, which has detected the measured light from the sternum portion of the cancer patient before administration of the drug and after administration of the drug; and from the obtained detected signal, the computation/storage apparatus 3 calculates the absolute hemoglobin concentration in the sternum portion of the cancer patient before administration of the drug and the absolute hemoglobin concentration in the sternum portion of the cancer patient after administration of the drug by time resolved spectroscopy, phase modulation spectroscopy, or the like, respectively, to obtain the data. Furthermore, from the data of the absolute hemoglobin concentration in the sternum portion of the cancer patient before administration of the drug and after administration of the drug, the computation/storage apparatus 3 generates the data of the first absolute hemoglobin concentration and the data of the second absolute hemoglobin concentration, and compares the first absolute hemoglobin concentration and the second absolute hemoglobin concentration. To the computation/storage apparatus 3, the input device 4 such as a keyboard and a mouse and the display device 5 such as a display are connected. The computation/storage apparatus 3 is a personal computer; a microcomputer; a smart device such as a smartphone or a tablet terminal; or a computer such as a cloud server to which the smart device is connected through a network. In the case where the computation/storage apparatus 3 is a smart device, the input device 4 and the display device 5 are built in the smart device. Moreover, in the case where the computation/storage apparatus 3 is a cloud server, the input device 4 and the display device 5 may be built in a smart device, or may be electrically connected to a personal computer.

In the case of the measurement by time resolved spectroscopy, the light source 6 outputs pulsed light as the measured light through the control of the computation/storage apparatus 3. Moreover, in the case of the measurement by phase modulation spectroscopy, the light source 6 outputs modulated light as the measured light through the control of the computation/storage apparatus 3. In the case of the measurement by time resolved spectroscopy, the light source 6 may output pulsed light having a plurality of wavelengths, or the pulsed light may be near-infrared light. The light source 6 may be built in the probe 8. In this case, the light source 6 serves as part of a light input unit 9 of the probe 8. Examples of the light source 6 include laser diodes, light emitting diodes, super luminescent diodes, and lamp-type light sources.

The photodetector 7 detects the measured light (such as scattered light) which is output from the light source 6 and passes through a measurement region 14 of a sternum portion 13, and outputs the detected signals. Specifically, the photodetector 7 detects the measured light (such as scattered light) which is input by the light input unit 9 in the probe 8 and passes through the measurement region 14 of the sternum portion 13, and outputs the detected signal to the computation/storage apparatus 3. The photodetector 7 may be built in the probe 8, and in this case, the photodetector 7 serves as part of a light output unit 10 in the probe 8. Examples of the photodetector include photomultiplier tubes, photodiodes such as avalanche photodiodes, and SiPM (Silicon Photomultipliers) such as MPPC (Multi-Pixel Photon Counter).

The probe 8 is composed of the light input unit 9, which inputs the measured light into the sternum portion 13, the measured light being output from the light source 6 and guided by a light guiding unit 11 such as optical fibers, and the light output unit 10 which outputs the measured light (such as scattered light) passing through the measurement region 14 of the sternum portion 13 via the light guiding unit 11 such as optical fibers into the photodetector 7. The measurement of the hemoglobin concentration is performed in the state where the probe 8 is disposed on the surface of a skin 12 above the sternum portion 13. The probe 8 may have the light source 6 and/or the photodetector 7 built therein. In this case, the light source 6 and/or the photodetector 7 built in the probe 8 may be electrically or wirelessly connected to the computation/storage apparatus 3.

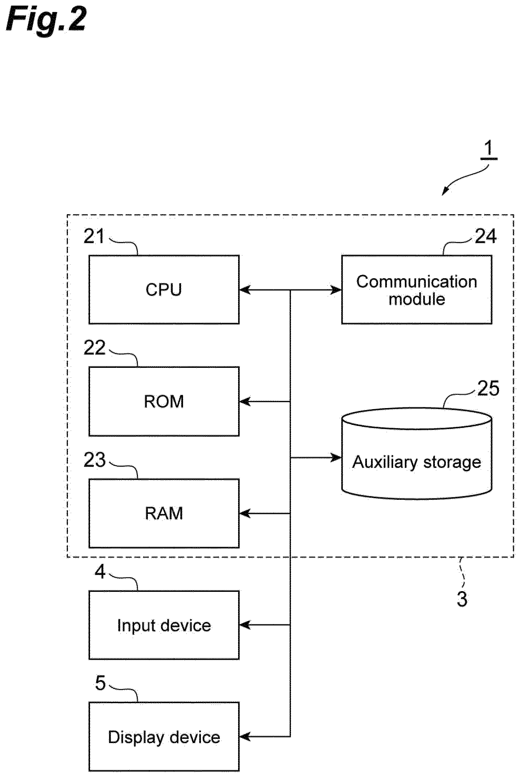

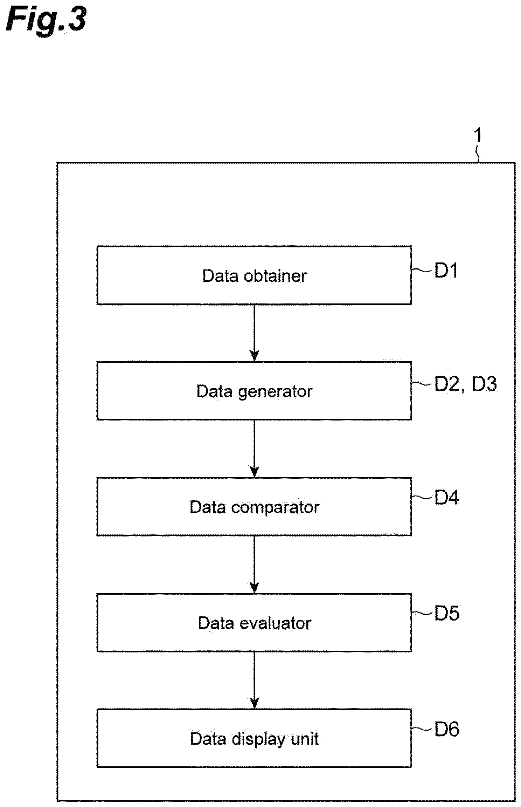

Next, the evaluation device will be described. Figure 2 is a schematic diagram illustrating the hardware-like configuration of the therapeutic effect evaluation device 1 according to one embodiment. Figure 3 is a schematic diagram illustrating a functional configuration of the therapeutic effect evaluation device 1 according to one embodiment.

As illustrated in Figure 2, the evaluation device 1 is physically composed as a computer including the computation/storage apparatus 3 composed of a CPU 21, a ROM 22, a RAM 23, and a communication module 24, such as a network card for performing communication with other devices such as the light source, the photodetector, the probe, and the like; a main storage such as an auxiliary storage 25 such as a hard disk, the input device 4 such as a keyboard and a mouse; and the display device 5 such as a display. The functions of the therapeutic effect evaluation device described later are implemented by operating the input device 4, the display device 5, and the communication module 24 under the control of the CPU 21 through reading of predetermined computer software onto hardware such as the CPU 21, the ROM 22, the RAM 23, and the like, and at the same time by performing reading and writing of data in the main storages 22 and 23 and the auxiliary storage 25.

As illustrated in Figure 3, the evaluation device 1 includes a data obtainer D1, a first data generator D2, a second data generator D3, and a data comparator D4 as functional components. The evaluation device 1 may further include a data evaluator D5, and a data display unit D6. The evaluation device 1 may further include a controller D11.

The data obtainer D1 obtains the detected signals output from the photodetector 7, which has detected the measured light from the sternum portion of the cancer patient before administration of the drug and after administration of the drug. From the obtained detected signal, the data obtainer D1 then calculates the absolute hemoglobin concentration in the sternum portion of the cancer patient before administration of the drug and the absolute hemoglobin concentration in the sternum portion of the cancer patient after administration of the drug by time resolved spectroscopy, phase modulation spectroscopy, or the like, respectively, to obtain the data. The data obtainer D1 may be a device which obtains the data of the absolute hemoglobin concentration calculated in the photodetector from the photodetector, or may be a device which obtains the data of the absolute hemoglobin concentration directly input from the input device. The data of the absolute hemoglobin concentration obtained by the data obtainer D1 may be stored in the data storage unit such as the auxiliary storage 25.

The first data generator D2 generates the data of the first absolute hemoglobin concentration from the data of the absolute hemoglobin concentration in the sternum portion of the cancer patient before administration of the drug. In the case where the number of pieces of data of the absolute hemoglobin concentration in the sternum portion of the cancer patient before administration of the drug obtained by the data obtainer D1 is one, the first data generator D2 generates the one piece of data as the data of the first absolute hemoglobin concentration. In contrast, in the case where the number of pieces of data of the absolute hemoglobin concentration in the sternum portion of the cancer patient before administration of the drug obtained by the data obtainer D1 is plural, the first data generator D2 generates a statistical value such as the average or median of the plurality of pieces of data as the data of the first absolute hemoglobin concentration. The first data generator D3 may read the data of the absolute hemoglobin concentration stored in the data storage unit such as the auxiliary storage 25, and may use it.

The second data generator D3 generates the data of the second absolute hemoglobin concentration from the data of the absolute hemoglobin concentration in the sternum portion of the cancer patient after administration of the drug. In the case where the number of pieces of data of the absolute hemoglobin concentration in the sternum portion of the cancer patient after administration of the drug obtained by the data obtainer D1 is one, the second data generator D3 generates the one piece of data as the data of the second absolute hemoglobin concentration. In contrast, in the case where the number of pieces of data of the absolute hemoglobin concentration in the sternum portion of the cancer patient after administration of the drug obtained by the data obtainer D1 is plural, the second data generator D3 generates a statistical value such as the average or median of the plurality of pieces of data as the data of the second absolute hemoglobin concentration. The second data generator D3 may read the data of the absolute hemoglobin concentration stored in the data storage unit such as the auxiliary storage 25, and may use it.

The first data generator D2 and the second data generator D3 may be the same data generator. The data of the first absolute hemoglobin concentration generated by the first data generator D2 and/or the data of the second absolute hemoglobin concentration generated by the second data generator D3 may be stored in the data storage unit such as the auxiliary storage 25.

The data comparator D4 compares the data of the first absolute hemoglobin concentration and the data of the second absolute hemoglobin concentration. The data comparator D4 may read the data of the absolute hemoglobin concentration stored in the data storage unit such as the auxiliary storage 25, and may use it.

The data evaluator D5 evaluates the therapeutic effect of the drug in the cancer patient based on the result of comparison performed by the data comparator D4. Moreover, the data evaluator D5 generates the evaluation data based on the result of evaluation. The data display unit D6 displays the evaluation data generated by the data evaluator D5. The data evaluator D5 may provide the evaluation data through a network or the like to the cancer patient, a medical profession, or a medical institution.

The evaluation device 1 may further include a data obtainer D7. The data obtainer D7 obtains the detected signals output from the photodetector 7, which has detected the measured light from the lesion site of the cancer patient before administration of the drug and after administration of the drug. From the obtained detected signal, the data obtainer D7 calculates the absolute hemoglobin concentration in the lesion site of the cancer patient before administration of the drug and the absolute hemoglobin concentration in the lesion site of the cancer patient after administration of the drug by time resolved spectroscopy, phase modulation spectroscopy, or the like, respectively, to obtain the data. The data obtainer D7 may be a device which obtains the data of the absolute hemoglobin concentration calculated in the photodetector from the photodetector, or may be a device which obtains the data of the absolute hemoglobin concentration directly input from the input device. The data of the absolute hemoglobin concentration obtained by the data obtainer D7 may be stored in the data storage unit such as the auxiliary storage 25. The data obtainer D7 may be the same data obtainer as the data obtainer D1.

The evaluation device 1 may further include a third data generator D8. The third data generator D8 generates the data of the third absolute hemoglobin concentration from the data of the absolute hemoglobin concentration in the lesion site of the cancer patient before administration of the drug. In the case where the number of pieces of data of the absolute hemoglobin concentration in the lesion site of the cancer patient before administration of the drug obtained by the data obtainer D7 is one, the third data generator D8 generates the one piece of data as the data of the third absolute hemoglobin concentration. In contrast, in the case where the number of pieces of data of the absolute hemoglobin concentration in the lesion site of the cancer patient before administration of the drug obtained by the data obtainer D7 is plural, the third data generator D8 generates a statistical value such as the average or median of the plurality of pieces of data as the data of the first absolute hemoglobin concentration. The third data generator D8 may read the data of the absolute hemoglobin concentration stored in the data storage unit such as the auxiliary storage 25, and may use it.

The evaluation device 1 may further include a fourth data generator D9. The fourth data generator D9 generates the data of the second absolute hemoglobin concentration from the data of the absolute hemoglobin concentration in the lesion site of the cancer patient after administration of the drug. In the case where the number of pieces of data of the absolute hemoglobin concentration in the lesion site of the cancer patient after administration of the drug obtained by the data obtainer D7 is one, the fourth data generator D9 generates the one piece of data as the data of the fourth absolute hemoglobin concentration. In contrast, in the case where the number of pieces of data of the absolute hemoglobin concentration in the lesion site of the cancer patient after administration of the drug obtained by the data obtainer D7 is plural, the fourth data generator D9 generates a statistical value such as the average or median of the plurality of pieces of data as the data of the fourth absolute hemoglobin concentration. The fourth data generator D9 may read the data of the absolute hemoglobin concentration stored in the data storage unit such as the auxiliary storage 25, and may use it.

The third data generator D8 and the fourth data generator D9 may be the same data generator. Moreover, the third data generator D8 and the fourth data generator D9 may be the same data generator as the first data generator D2 and the second data generator D3. The data of the third absolute hemoglobin concentration generated by the third data generator D8 and/or the data of the fourth absolute hemoglobin concentration generated by the fourth data generator D9 may be stored in the auxiliary storage 25 or the like.

The evaluation device 1 may further include a data comparator D10. The data comparator D10 compares the data of the third absolute hemoglobin concentration and the data of the fourth absolute hemoglobin concentration. The data comparator D10 may read the data of the absolute hemoglobin concentration stored in the data storage unit such as the auxiliary storage 25, and may use it.

In the case where the computation/storage apparatus 3 is connected to the light source 6, the evaluation device 1 may further include a controller D11 which controls the output of the measured light from the light source 6.

### [Therapeutic effect evaluation program of drug in cancer patient]

The therapeutic effect evaluation program according to the present embodiment causes a computer to execute: data obtaining processing to obtain data of an absolute hemoglobin concentration in the sternum portion of the cancer patient before administration of the drug and data of an absolute hemoglobin concentration in the sternum portion of the cancer patient after administration of the drug; data generation processing to generate data of a first absolute hemoglobin concentration from the data of the absolute hemoglobin concentration in the sternum portion of the cancer patient before administration of the drug obtained by the data obtaining processing, and to generate data of a second absolute hemoglobin concentration from the data of the absolute hemoglobin concentration in the sternum portion of the cancer patient after administration of the drug obtained by the data obtaining processing; and the data comparison processing to compare the data of the first absolute hemoglobin concentration and the data of the second absolute hemoglobin concentration. Accordingly, the therapeutic effect evaluation program according to the present embodiment causes the computer to function as the data obtainer D1, the first data generator D2, the second data generator D3, and the data comparator D4 described above. The therapeutic effect evaluation program may further cause the computer to function as the data evaluator D5 and the data display unit D6 described above. The therapeutic effect evaluation program may further cause the computer to function as the data obtainer D7, the third data generator D8, the fourth data generator D9, and the data comparator D10 described above. The therapeutic effect evaluation program may further cause the computer to function as the controller D11 described above. The computer operates as a therapeutic effect evaluation device by reading the therapeutic effect evaluation program into the computer. The therapeutic effect evaluation program may be recorded in a computer-readable recording medium, for example, and may be provided. The recording medium may be a non-temporary recording medium. Examples of the recording medium include flexible disks, recording media such as CDs and DVDs, recording media such as ROMs, and semiconductor memories.

### [Method of evaluating therapeutic effect of drug performed by evaluation device]

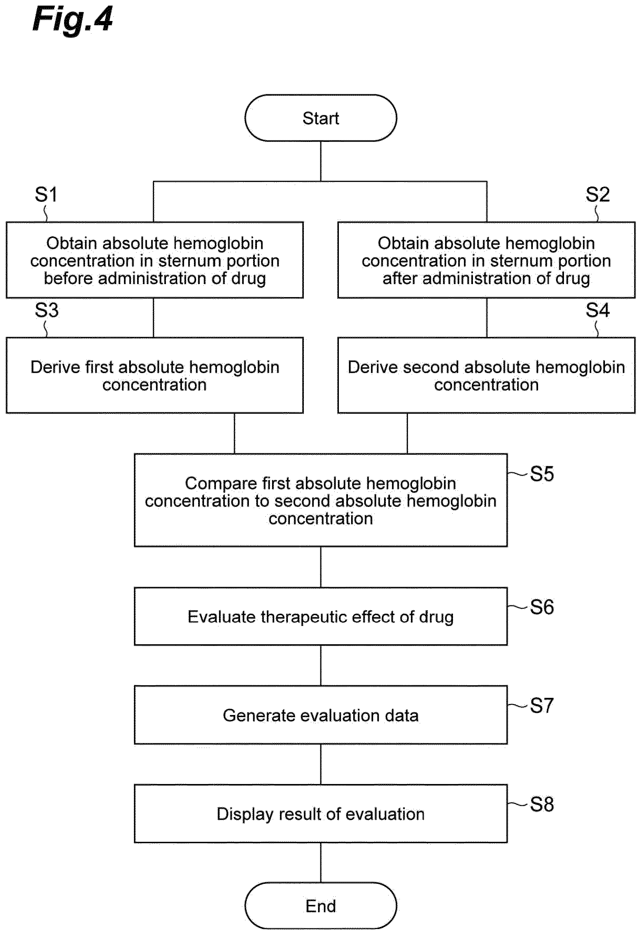

Figure 4 is a flowchart of the evaluation method according to one embodiment. By the method of evaluating the therapeutic effect of the drug performed by the evaluation device 1, the evaluation of the therapeutic effect of the drug in the cancer patient can be automatically preformed with high precision.

First, the data obtainer D1 obtains the detected signal output from a photodetector, which has detected the measured light from the sternum portion of the cancer patient before administration of the drug. The data obtainer D1 then calculates the absolute hemoglobin concentration in the sternum portion of the cancer patient before administration of the drug from the obtained detected signal by time resolved spectroscopy, phase modulation spectroscopy, or the like to obtain the data (obtaining step S1). Next, the data obtainer D1 obtains the detected signal output from the photodetector, which has detected the measured light from the sternum portion of the cancer patient after administration of the drug. The data obtainer D1 then calculates the absolute hemoglobin concentration in the sternum portion of the cancer patient after administration of the drug from the obtained detected signal by time resolved spectroscopy, phase modulation spectroscopy, or the like to obtain the data (obtaining step S2). The obtaining steps S1 and S2 may be a step of obtaining data of the absolute hemoglobin concentration calculated in the photodetector from the photodetector, or may be a step of obtaining data of the absolute hemoglobin concentration directly input from the input device. The obtaining steps S1 and S2 may comprise storing the obtained data of the absolute hemoglobin concentration in the data storage unit such as the auxiliary storage 25. The obtaining steps S1 and S2 may be performed at the same time, or may be performed at different timings.

Next, the first data generator D2 generates the data of the first absolute hemoglobin concentration from the data of the absolute hemoglobin concentration in the sternum portion of the cancer patient before administration of the drug obtained in the obtaining step S1 (generating step S3). In the case where the number of pieces of data of the absolute hemoglobin concentration in the sternum portion of the cancer patient before administration of the drug obtained in the obtaining step S1 is one, the first data generator D2 generates the one piece of data as the data of the first absolute hemoglobin concentration. In contrast, in the case where the number of pieces of data of the absolute hemoglobin concentration in the sternum portion of the cancer patient before administration of the drug obtained in the obtaining step S1 is plural, the first data generator D2 generates a statistical value of the average or median of the plurality of pieces of data as the data of the first absolute hemoglobin concentration. The generating step S3 may comprise reading the data of the absolute hemoglobin concentration stored in the data storage unit such as the auxiliary storage 25.

Next, the second data generator D3 generates the data of the second absolute hemoglobin concentration from the data of the absolute hemoglobin concentration in the sternum portion of the cancer patient after administration of the drug obtained in the obtaining step S2 (generating step S4). In the case where the number of pieces of data of the absolute hemoglobin concentration in the sternum portion of the cancer patient after administration of the drug obtained in the obtaining step S2 is one, the second data generator D3 generates the one piece of data as the data of the second absolute hemoglobin concentration. In contrast, in the case where the number of pieces of data of the absolute hemoglobin concentration in the sternum portion of the cancer patient after administration of the drug obtained in the obtaining step S2 is plural, the second data generator D3 generates a statistical value of the average or median of the plurality of pieces of data as the data of the second absolute hemoglobin concentration. The generating step S4 may comprise reading the data of the absolute hemoglobin concentration stored in the data storage unit such as the auxiliary storage 25.

The generating steps S3 and S4 may be performed at the same time, or may be performed at different timings. Moreover, the generating steps S3 and S4 may comprise storing the generated data of the third absolute hemoglobin concentration and/or the generated data of the fourth absolute hemoglobin concentration in the data storage unit such as the auxiliary storage 25.

Next, the data comparator D4 compares the data of the first absolute hemoglobin concentration and the data of the second absolute hemoglobin concentration (comparison step S5). The comparison step S5 may comprise reading the data of the absolute hemoglobin concentration stored in the data storage unit such as the auxiliary storage 25.

Next, the data evaluator D5 evaluates the therapeutic effect of the drug in the cancer patient based on the result of comparison performed through the comparison step S5 (evaluation step S6). Specifically, the evaluation is performed by the method described in [Method of evaluating therapeutic effect of drug in cancer patient]. The data evaluator D5 generates the evaluation data for evaluating the therapeutic effect of the drug in the cancer patient, based on the result of evaluation performed through the evaluation step S5 (generation step S7).

Next, the data display unit D6 displays the result of evaluation performed in the evaluation step S6 (display step S8). For example, the evaluation data generated through the generation step S7 is displayed by the data display unit D6.

### Examples

Hereinafter, the embodiments will be more specifically described based on Examples. It should be noted that the embodiments are not limited to these Examples.

### [Example 1: measurement of absolute hemoglobin concentration in the lesion site and sternum portion of breast cancer patient]

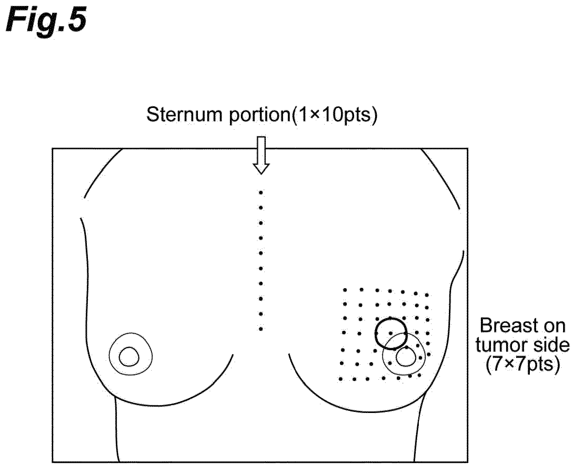

In the sternum portions of 69 primary breast cancer patients, the absolute hemoglobin concentrations in 10 different sites illustrated in Figure 5 before and after administration of an anticancer agent were measured with a time resolved spectroscope (TRS-21, made by Hamamatsu Photonics K.K.) using near-infrared light at three wavelengths (760 nm, 800 nm, and 830 nm) as measured light, and the average was calculated. For the absolute hemoglobin concentration after administration of the anticancer agent, the absolute hemoglobin concentrations after cycle 1 (the 21th day after administration of the anticancer agent) and after cycle 2 (the 21th day after administration of the anticancer agent) were measured.

Moreover, in the lesion sites (breast on the tumor side) of the 69 primary breast cancer patients, the absolute hemoglobin concentrations at 49 different sites illustrated in Figure 5 before and after administration of the anticancer agent were measured by the same method as described above.

The averages of the absolute hemoglobin concentrations before and after administration of the anticancer agent in the sternum portions and the lesion sites of the breast cancer patients (14 patients), who were determined after the end of chemotherapy (about 6 months) that there was a pathologically therapeutic effect of the anticancer agent, and the averages of the absolute hemoglobin concentrations before and after administration of the anticancer agent in the sternum portions and lesion sites of the breast cancer patients (52 patients), who were determined that there was no pathologically therapeutic effect of the anticancer agent are shown in Table 1.

**[Table 1]**

| | Average of absolute hemoglobin concentrations before administration of anticancer agent (µM) | Average of absolute hemoglobin concentrations after first administration of anticancer agent (µM) | Average of absolute hemoglobin concentrations after second administration of anticancer agent (µM) |
|---|---|---|---|
| Sternum portion (with therapeutic effect) | 33.4 | 30.8 | 31.0 |
| Sternum portion (no therapeutic effect) | 32.3 | 32.3 | 31.8 |
| Lesion site (with therapeutic effect) | 39.9 | 30.0 | 26.6 |
| Lesion site (no therapeutic effect) | 38.9 | 32.2 | 30.5 |

In the breast cancer patients exhibiting the therapeutic effect, the absolute hemoglobin concentrations in the sternum portions and the lesion sites after administration of the anticancer agent both reduced compared to those before administration of the anticancer agent. In contrast, in the breast cancer patients not exhibiting the therapeutic effect, the absolute hemoglobin concentrations in the sternum portions after administration of the anticancer agent barely changed compared to those before administration of the anticancer agent. Moreover, in the breast cancer patients not exhibiting the therapeutic effect, although the absolute hemoglobin concentrations in the lesion sites after administration of the anticancer agent reduced compared to those before administration of the anticancer agent, the reduction rate was smaller than that in the sternum portions.

From above, it was verified that it can be evaluated that there is a therapeutic effect of the drug if the absolute hemoglobin concentration in the sternum portion of the cancer patient after administration of the drug is lower than the absolute hemoglobin concentration before administration of the drug.

### [Reference Example 1: comparison using ROC curve (Receiver Operating Characteristic Curve) of pCR (pathological complete response) predicted diagnostic ability by measurement of absolute hemoglobin concentration in lesion site and sternum portion of breast cancer patient]

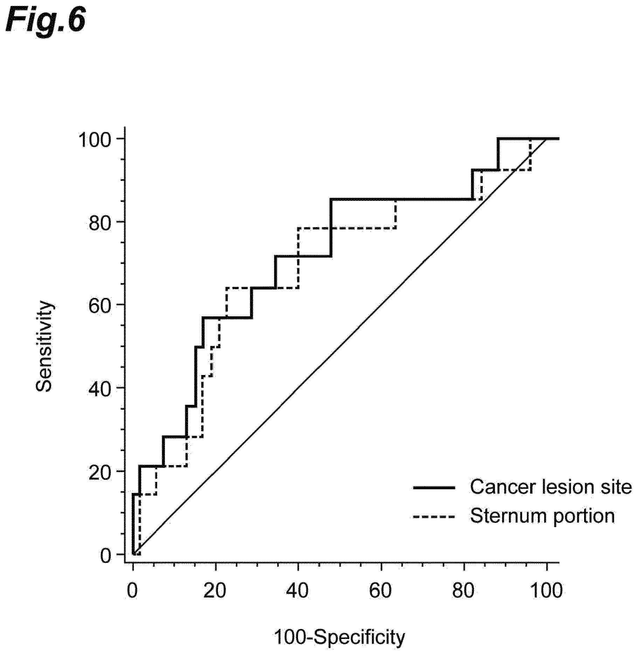

In the breast cancer patients (14 patients) who were determined that there was a therapeutic effect of the anticancer agent and the breast cancer patients (52 patients) who were determined that there was no therapeutic effect of the anticancer agent in Example 1, ROC curves where the absolute hemoglobin concentrations in the lesion sites and the sternum portions measured after cycle 2 (on the 21th day after administration of the anticancer agent) were used in pCR predictive diagnosis were created by plotting 100-specificity (Specificity) as the abscissa against sensitivity (Sensitivity) as the ordinate (Figure 6). Here, the specificity indicates the rate where the patient exhibiting no therapeutic effect is evaluated from the comparison of the absolute hemoglobin concentration as that there is no therapeutic effect. The sensitivity indicates the rate where the patient exhibiting the therapeutic effect is evaluated from the comparison of the absolute hemoglobin concentration as that there is a therapeutic effect. An ROC curve toward the upper left direction farther away from the straight line connecting the origin to the point of 100 on the abscissa and the ordinate, namely, an ROC area under the curve (AUC) close to 1 indicates a higher predicted diagnostic ability.

The AUCs in the case of pCR predictive diagnosis from the absolute hemoglobin concentrations in the lesion sites and the sternum portions were 0.713 and 0.681, respectively. It was verified that the measurement in the sternum portion has the pCR predicted diagnostic ability similar to that of the measurement in the lesion site.

### [Reference Example 2: comparison using ROC curve of pCR predicted diagnostic ability by measurement of absolute hemoglobin concentration in sternum portion of breast cancer patient after cycle 1 and after cycle 2 of administration of anticancer agent]

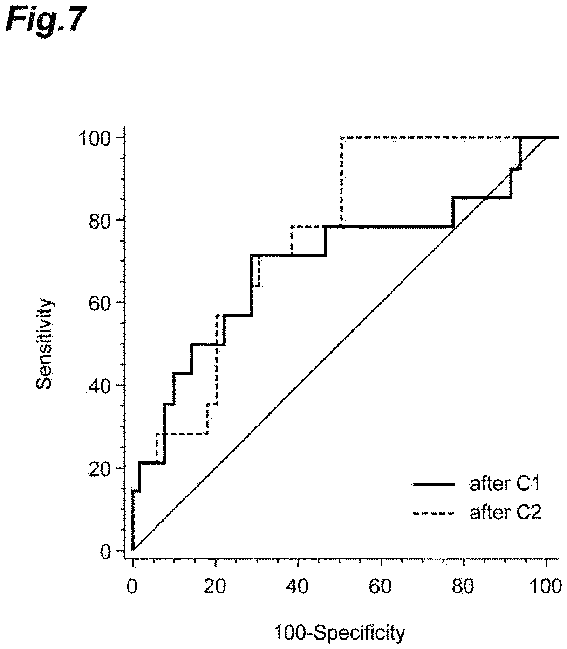

In accordance with the method described in Reference Example 1, ROC curves where the absolute hemoglobin concentrations in the sternum portions measured after cycle 1 (on the 21th day after administration of the anticancer agent) and after cycle 2 (on the 21th day after administration of the anticancer agent) were used in the pCR predictive diagnosis were created (Figure 7).

The AUCs in the case of pCR predictive diagnosis from the absolute hemoglobin concentrations after cycle 1 and after cycle 2 were 0.69 and 0.75, respectively. It was verified that the pCR predicted diagnostic ability by measurement after cycle 2 is more significantly improved.

### Reference Signs List

1...evaluation device, 2...optical measuring apparatus, 3...computation/storage apparatus (computer), 4...input device, 5...display device, 6...light source, 7...photodetector, 8...probe, 9...light input unit, 10...light output unit, 11...light guiding unit, 12...skin, 13...sternum portion (sternum site), 14...measurement region, 21...CPU, 22...ROM, 23...RAM, 24...communication module, 25...auxiliary storage (data storage unit), D1...data obtainer, D2...first data generator, D3...second data generator, D4...data comparator, D5...data evaluator, D6...data display unit, S1...first obtaining step, S2...second obtaining step, S3...first generating step, S4...second generating step, S5...comparison step, S6...evaluation step, S7...generation step, 58...display step.

## Claims

1. An evaluation device of a therapeutic effect of a drug in a cancer patient, the evaluation device comprising:
a data obtainer configured to obtain data of an absolute hemoglobin concentration in a sternum portion of the cancer patient before administration of the drug and data of an absolute hemoglobin concentration in the sternum portion of the cancer patient after administration of the drug, wherein absolute hemoglobin concentration refers to the absolute value of the concentration of oxygenated hemoglobin, or the absolute value of the concentration of deoxygenated hemoglobin, or the total value of the absolute value of the concentration of oxygenated hemoglobin and the absolute value of the concentration of deoxygenated hemoglobin;
a data generator configured to generate data of a first absolute hemoglobin concentration from the data of the absolute hemoglobin concentration in the sternum portion of the cancer patient before administration of the drug obtained by the data obtainer, and generating data of a second absolute hemoglobin concentration from the data of the absolute hemoglobin concentration in the sternum portion of the cancer patient after administration of the drug obtained by the data obtainer;
a data comparator configured to compare the data of the first absolute hemoglobin concentration and the data of the second absolute hemoglobin concentration; and.
a data evaluator configured to evaluate the therapeutic effect of the drug in the cancer patient based on a result of comparison performed by the data comparator.

2. The evaluation device of a therapeutic effect of a drug in a cancer patient according to claim 1, wherein the data evaluator evaluates that there is a therapeutic effect of the drug if the second absolute hemoglobin concentration is lower than the first absolute hemoglobin concentration.

3. The evaluation device of a therapeutic effect of a drug in a cancer patient according to claim 1 or 2, wherein the data evaluator evaluates that there is no therapeutic effect of the drug if the second absolute hemoglobin concentration is equal to or higher than the first absolute hemoglobin concentration.

4. The evaluation device of a therapeutic effect of a drug in a cancer patient according to any one of claims 1 to 3, wherein the data generator is further configured to generate a statistical value of data of a plurality of absolute hemoglobin concentrations as the data of the first absolute hemoglobin concentration and/or generate a statistical value of data of a plurality of absolute hemoglobin concentrations as the data of the second absolute hemoglobin concentration.

5. The evaluation device of a therapeutic effect of a drug in a cancer patient according to claim 1, wherein
the data obtainer is further configured to obtain data of an absolute hemoglobin concentration in a lesion site of the cancer patient before administration of the drug and data of an absolute hemoglobin concentration in the lesion site of the cancer patient after administration of the drug;
the data generator is further configured to generate data of a third absolute hemoglobin concentration from the data of the absolute hemoglobin concentration in the lesion site of the cancer patient before administration of the drug obtained by the data obtainer, and further to generate data of a fourth absolute hemoglobin concentration from the data of the absolute hemoglobin concentration in the lesion site of the cancer patient after administration of the drug obtained by the data obtainer; and
the data comparator is further configured to compare the data of the third absolute hemoglobin concentration and the data of the fourth absolute hemoglobin concentration.

6. The evaluation device of a therapeutic effect of a drug in a cancer patient according to claim 5, wherein the data evaluator evaluates that there is a therapeutic effect of the drug in the cancer patient if the second absolute hemoglobin concentration is lower than the first absolute hemoglobin concentration and the fourth absolute hemoglobin concentration is lower than the third absolute hemoglobin concentration.

7. The evaluation device of a therapeutic effect of a drug in a cancer patient according to any one of claims 1 to 6, wherein the cancer patient is a breast cancer patient.

8. An evaluation program for evaluating a therapeutic effect of a drug in a cancer patient, the program causing a computer to execute:
data obtaining processing to obtain data of an absolute hemoglobin concentration in a sternum portion of the cancer patient before administration of the drug and data of an absolute hemoglobin concentration in the sternum portion of the cancer patient after administration of the drug, wherein absolute hemoglobin concentration refers to the absolute value of the concentration of oxygenated hemoglobin, or the absolute value of the concentration of deoxygenated hemoglobin, or the total value of the absolute value of the concentration of oxygenated hemoglobin and the absolute value of the concentration of deoxygenated hemoglobin;
data generation processing to generate data of a first absolute hemoglobin concentration from the data of the absolute hemoglobin concentration in the sternum portion of the cancer patient before administration of the drug obtained by the data obtaining processing, and to generate data of a second absolute hemoglobin concentration from the data of the absolute hemoglobin concentration in the sternum portion of the cancer patient after administration of the drug obtained by the data obtaining processing;
data comparison processing to compare the data of the first absolute hemoglobin concentration and the data of the second absolute hemoglobin concentration; and
data evaluation to evaluate the therapeutic effect of the drug in the cancer patient based on a result of comparison performed by the data comparator.

## Patentansprüche

1. Vorrichtung zur Auswertung einer therapeutischen Wirkung eines Arzneimittels bei einem Krebspatienten, wobei die Vorrichtung umfasst:
eine Datenerfassungseinheit, konfiguriert zum Erfassen von Daten einer absoluten Hämoglobinkonzentration in einem Sternumbereich des Krebspatienten vor Verabreichung des Arzneimittels und von Daten einer absoluten Hämoglobinkonzentration im Sternumbereich des Krebspatienten nach Verabreichung des Arzneimittels, wobei absolute Hämoglobinkonzentration sich auf den absoluten Wert der Konzentration an oxygeniertem Hämoglobin oder auf den absoluten Wert der Konzentration an desoxygeniertem Hämoglobin oder auf den Gesamtwert des absoluten Wertes der Konzentration an oxygeniertem Hämoglobin und des absoluten Wertes der Konzentration an desoxygeniertem Hämoglobin bezieht;
eine Datengenerierungseinheit, konfiguriert zum Generieren von Daten einer ersten absoluten Hämoglobinkonzentration aus den Daten der absoluten Hämoglobinkonzentration im Sternumbereich des Krebspatienten vor Verabreichung des Arzneimittels, erfasst durch die Datenerfassungseinheit, und Generieren von Daten einer zweiten absoluten Hämoglobinkonzentration aus den Daten der absoluten Hämoglobinkonzentration im Sternumbereich des Krebspatienten nach Verabreichung des Arzneimittels, erfasst durch die Datenerfassungseinheit;
eine Datenvergleichseinheit, konfiguriert zum Vergleichen der Daten der ersten absoluten Hämoglobinkonzentration und der Daten der zweiten absoluten Hämoglobinkonzentration; und
eine Datenauswertungseinheit, konfiguriert zum Auswerten der therapeutischen Wirkung des Arzneimittels im Krebspatienten, basierend auf einem Ergebnis eines durch die Datenvergleichseinheit durchgeführten Vergleichs.

2. Vorrichtung zum Auswerten einer therapeutischen Wirkung eines Arzneimittels bei einem Krebspatienten gemäß Anspruch 1, wobei die Datenauswertungseinheit auswertet, dass eine therapeutische Wirkung des Arzneimittels vorliegt, wenn die zweite absolute Hämoglobinkonzentration niedriger als die erste absolute Hämoglobinkonzentration ist.

3. Vorrichtung zum Auswerten einer therapeutischen Wirkung eines Arzneimittels bei einem Krebspatienten gemäß Anspruch 1 oder 2, wobei die Datenauswertungsreinheit auswertet, dass keine therapeutische Wirkung des Arzneimittels vorliegt, wenn die zweite absolute Hämoglobinkonzentration gleich oder höher als die erste absolute Hämoglobinkonzentration ist.

4. Vorrichtung zum Auswerten einer therapeutischen Wirkung eines Arzneimittels bei einem Krebspatienten gemäß einem der Ansprüche 1 bis 3, wobei die Datengenerierungseinheit weiterhin konfiguriert ist, einen statistischen Datenwert einer Vielzahl absoluter Hämoglobinkonzentrationen als die Daten der ersten absoluten Hämoglobinkonzentration und/oder einen statistischen Datenwert einer Mehrzahl absoluter Hämoglobinkonzentrationen als die Daten der zweiten absoluten Hämoglobinkonzentration zu generieren.

5. Vorrichtung zum Auswerten einer therapeutischen Wirkung eines Arzneimittels bei einem Krebspatienten gemäß Anspruch 1, wobei
die Datenerfassungseinheit weiterhin konfiguriert ist, Daten einer absoluten Hämoglobinkonzentration in einem Läsionsbereich des Krebspatienten vor Verabreichung des Arzneimittels und Daten einer absoluten Hämoglobinkonzentration in dem Läsionsbereich des Krebspatienten nach Verabreichung des Wirkstoffs zu erfassen;
die Datengenerierungseinheit weiterhin konfiguriert ist, Daten einer dritten absoluten Hämoglobinkonzentration aus den Daten der absoluten Hämoglobinkonzentration im Läsionsbereich des Krebspatienten vor Verabreichung des Arzneimittels, erfasst durch die Datenerfassungseinheit, zu generieren und weiterhin Daten einer vierten absoluten Hämoglobinkonzentration aus den Daten der absoluten Hämoglobinkonzentration im Läsionsbereich des Krebspatienten nach Verabreichung des Arzneimittels, erfasst durch die Datenerfassungseinheit, zu generieren; und
die Datenvergleichseinheit weiterhin konfiguriert ist, die Daten der dritten absoluten Hämoglobinkonzentration und die Daten der vierten absoluten Hämoglobinkonzentration zu vergleichen.

6. Vorrichtung zum Auswerten einer therapeutischen Wirkung eines Arzneimittels bei einem Krebspatienten gemäß Anspruch 5, wobei die Datenauswertungseinheit auswertet, dass eine therapeutische Wirkung des Arzneimittels beim Krebspatienten vorliegt, wenn die zweite absolute Hämoglobinkonzentration niedriger als die erste absolute Hämoglobinkonzentration ist und die vierte absolute Hämoglobinkonzentration niedriger als die dritte absolute Hämoglobinkonzentration ist.

7. Vorrichtung zum Auswerten einer therapeutischen Wirkung eines Arzneimittels bei einem Krebspatienten gemäß einem der Ansprüche 1 bis 6, wobei der Krebspatient ein Brustkrebspatient ist.

8. Programm zum Evaluieren einer therapeutischen Wirkung eines Arzneimittels bei einem Krebspatienten, wobei das Programm bewirkt, dass ein Computer ausführt:
Datenerfassungsverarbeitung zum Erfassen von Daten einer absoluten Hämoglobinkonzentration in einem Sternumbereich des Krebspatienten vor Verabreichung des Arzneimittels und von Daten einer absoluten Hämoglobinkonzentration im Sternumbereich des Krebspatienten nach Verabreichung des Arzneimittels, wobei absolute Hämoglobinkonzentration sich auf den absoluten Wert der Konzentration an oxygeniertem Hämoglobin oder auf den absoluten Wert der Konzentration an desoxygeniertem Hämoglobin oder auf den Gesamtwert des absoluten Wertes der Konzentration an oxygeniertem Hämoglobin und des absoluten Wertes der Konzentration an desoxygeniertem Hämoglobin bezieht;
Datenerfassungsverarbeitung zum Generieren von Daten einer ersten absoluten Hämoglobinkonzentration aus den Daten der absoluten Hämoglobinkonzentration im Sternumbereich des Krebspatienten vor Verabreichung des Arzneimittels, erfasst durch die Datenerfassungsverarbeitung, und Generieren von Daten einer zweiten absoluten Hämoglobinkonzentration aus den Daten der absoluten Hämoglobinkonzentration im Sternumbereich des Krebspatienten nach Verabreichung des Arzneimittels, erfasst durch die Datenerfassungsverarbeitung;
eine Datenvergleichsverarbeitung zum Vergleichen der Daten der ersten absoluten Hämoglobinkonzentration und der Daten der zweiten absoluten Hämoglobinkonzentration; und
Datenauswertungsverarbeitung, konfiguriert zum Auswerten der therapeutischen Wirkung des Arzneimittels im Krebspatienten, basierend auf einem Ergebnis eines durch die Datenvergleichseinheit durchgeführten Vergleichs.

## Revendications

1. Dispositif d'évaluation d'un effet thérapeutique d'un médicament chez un patient cancéreux, le dispositif d'évaluation comprenant :
un obtenteur de données configuré pour obtenir des données d'une concentration absolue d'hémoglobine dans une partie du sternum du patient cancéreux avant l'administration du médicament et des données d'une concentration absolue d'hémoglobine dans la partie du sternum du patient cancéreux après l'administration du médicament, dans lequel la concentration absolue d'hémoglobine se réfère à la valeur absolue de la concentration d'hémoglobine oxygénée, ou la valeur absolue de la concentration d'hémoglobine désoxygénée, ou la valeur totale de la valeur absolue de la concentration d'hémoglobine oxygénée et la valeur absolue de la concentration d'hémoglobine désoxygénée ;
un générateur de données configuré pour générer des données d'une première concentration absolue d'hémoglobine à partir des données de la concentration absolue d'hémoglobine dans la partie du sternum du patient cancéreux avant l'administration du médicament obtenu par l'obtenteur de données, et générant des données d'une deuxième concentration absolue d'hémoglobine à partir des données de la concentration absolue d'hémoglobine dans la partie du sternum du patient cancéreux après l'administration du médicament obtenues par l'obtenteur de données ;
un comparateur de données configuré pour comparer les données de la première concentration absolue d'hémoglobine et les données de la deuxième concentration absolue d'hémoglobine ; et
un évaluateur de données configuré pour évaluer l'effet thérapeutique du médicament chez le patient cancéreux sur la base d'un résultat de comparaison réalisé par le comparateur de données.

2. Dispositif d'évaluation d'un effet thérapeutique d'un médicament chez un patient cancéreux selon la revendication 1, dans lequel l'évaluateur de données évalue qu'il y a un effet thérapeutique du médicament si la deuxième concentration absolue d'hémoglobine est inférieure à la première concentration absolue d'hémoglobine.

3. Dispositif d'évaluation d'un effet thérapeutique d'un médicament chez un patient cancéreux selon la revendication 1 ou 2, dans lequel l'évaluateur de données évalue qu'il n'y a pas d'effet thérapeutique du médicament si la deuxième concentration absolue d'hémoglobine est égale ou supérieure à la première concentration absolue d'hémoglobine.

4. Dispositif d'évaluation d'un effet thérapeutique d'un médicament chez un patient cancéreux selon l'une quelconque des revendications 1 à 3, dans lequel le générateur de données est en outre configuré pour générer une valeur statistique de données d'une pluralité de concentrations absolues d'hémoglobine comme les données de la première concentration absolue d'hémoglobine et/ou générer une valeur statistique de données d'une pluralité de concentrations absolues d'hémoglobine comme les données de la deuxième concentration absolue d'hémoglobine.

5. Dispositif d'évaluation d'un effet thérapeutique d'un médicament chez un patient cancéreux selon la revendication 1, dans lequel
l'obtenteur de données est en outre configuré pour obtenir des données d'une concentration absolue d'hémoglobine dans un site de lésion du patient cancéreux avant l'administration du médicament et des données d'une concentration absolue d'hémoglobine dans le site de lésion du patient cancéreux après l'administration du médicament ;
le générateur de données est en outre configuré pour générer des données d'une troisième concentration absolue d'hémoglobine à partir des données de la concentration absolue d'hémoglobine dans le site de lésion du patient cancéreux avant l'administration du médicament obtenu par l'obtenteur de données, et en outre pour générer des données d'une quatrième concentration absolue d'hémoglobine à partir des données de la concentration absolue d'hémoglobine dans le site de lésion du patient cancéreux après l'administration du médicament obtenu par l'obtenteur de données ; et
le comparateur de données est en outre configuré pour comparer les données de la troisième concentration absolue d'hémoglobine et les données de la quatrième concentration absolue d'hémoglobine.

6. Dispositif d'évaluation d'un effet thérapeutique d'un médicament chez un patient cancéreux selon la revendication 5, dans lequel l'évaluateur de données évalue qu'il y a un effet thérapeutique du médicament chez le patient cancéreux si la deuxième concentration absolue d'hémoglobine est inférieure à la première concentration absolue d'hémoglobine et la quatrième concentration absolue d'hémoglobine est inférieure à la troisième concentration absolue d'hémoglobine.

7. Dispositif d'évaluation d'un effet thérapeutique d'un médicament dans un patient cancéreux selon l'une quelconque des revendications 1 à 6, dans lequel le patient cancéreux est un patient atteint d'un cancer du sein.

8. Programme d'évaluation pour évaluer un effet thérapeutique d'un médicament chez un patient cancéreux, le programme amenant un ordinateur à exécuter :
un traitement d'obtention de données pour obtenir des données d'une concentration absolue d'hémoglobine dans une partie du sternum du patient cancéreux avant l'administration du médicament et des données d'une concentration absolue d'hémoglobine dans la partie du sternum du patient cancéreux après l'administration du médicament, dans lequel la concentration absolue d'hémoglobine se réfère à la valeur absolue de la concentration d'hémoglobine oxygénée, ou la valeur absolue de la concentration d'hémoglobine désoxygénée, ou la valeur totale de la valeur absolue de la concentration d'hémoglobine oxygénée et la valeur absolue de la concentration d'hémoglobine désoxygénée ;
un traitement de génération de données pour générer des données d'une première concentration absolue d'hémoglobine à partir des données de la concentration absolue d'hémoglobine dans la partie du sternum du patient cancéreux avant l'administration du médicament obtenues par le traitement d'obtention de données, et pour générer les données d'une deuxième concentration absolue d'hémoglobine à partir des données de la concentration absolue d'hémoglobine dans la partie du sternum du patient cancéreux après l'administration du médicament obtenues par le traitement d'obtention de données;
un traitement de comparaison de données pour comparer les données de la première concentration absolue d'hémoglobine et les données de la deuxième concentration absolue d'hémoglobine ; et
une évaluation de données pour évaluer l'effet thérapeutique du médicament chez le patient cancéreux sur la base d'un résultat de la comparaison réalisée par le comparateur de données.
